# EUROPÄISCHE PATENTANMELDUNG

(11) **EP 1 913 927 A1**
(43) Veröffentlichungstag der Anmeldung: **23.04.2008**
(21) Anmeldenummer: 06021969.8
(22) Anmeldetag: 19.10.2006
(51) Int. Cl.: A61K 6/027, A61K 6/083

(54) **Polymerisierbares Dentalmaterial mit Nanodiamant**

(71) Anmelder: Ernst Mühlbauer GmbH & Co.KG, 25870 Norderfriedrichskoog (DE)
(72) Erfinder: Lück, Rainer Dr.sc., 25436 Tornesch (DE); Krüger, Anke Dr., 24103 Kiel (DE)
(74) Vertreter: Glawe, Delfs, Moll

(57) **Zusammenfassung**

Die Erfindung verbessert die mechanischen Eigenschaften des Dentalmaterials, wie die Druckfestigkeit.

## Beschreibung

Die Erfindung betrifft ein polymerisierbares Dentalmaterial mit Nanodiamant.

Polymerisierbare Dentalmaterialien, bei denen eine organische Phase mit einer anorganischen Phase kombiniert ist, sind dem Fachmann als "Komposite" geläufig und beispielsweise ausführlich beschrieben in Ullmanns Encyclopaedia of Industrial Chemistry, 6. Auflage, Band 10, Seite 355 ff. (denture base resins). Die organische Phase besteht üblicherweise aus dentalen Harzen, zumeist Acrylaten und Methacrylaten, in denen die Komponenten des Initiatorsystems und ev. weitere Additive gelöst sind. Die anorganische Phase besteht üblicherweise aus Füllstoffen im Mikrometerbereich. Ebenfalls ist bekannt, Nanopartikel wie z.B. Aerosile, beispielsweise das unter den Handelsnamen Aerosil R972 vertriebene Produkt der Fa. Degussa, in Dentalkomposite einzuarbeiten um deren Handhabungseigenschaften einzustellen. Zumeist liegen die Aerosilpartikel dabei als größere Agglomerate oder Aggregate vor.

In WO-A-2005/097045 wird vorgeschlagen, Nanodiamant in einer Vielzahl von Produkten, unter anderem auch in Dentalmaterial, zu verwenden. WO-A-2005/097045 offenbart gemäß dem Beispiel 3 Dentalmaterial zur Verwendung als Füllungs- und Rekonstrüktionsmaterial, dass 30-70 Vol.-% Nanodiamant mit einer Größe von 6-8 nm, 0-10 Vol.-% Titaniumdioxidund 30-70 Vol.-% Methacrylatharz aufweist.

Der vorliegenden Erfindung liegt die Aufgabe zu Grunde ein polymerisierbares Dentalmaterial zu schaffen, dass sich durch gute mechanische Eigenschaften, wie eine hohe Druckfestigkeit auszeichnet.

Die Erfindung löst diese Aufgabe durch ein polymerisierbares Dentalmaterial mit den Merkmalen des Anspruchs 1.

Die Erfindung hat erkannt, dass Nanodiamant, - im Unterschied zu herkömmlichen Nanopartikeln, wie sie im Stand der Technik verwendet werden -, überraschend die Druckfestigkeit des polymerisierbaren Dentalmaterials in hohem Maße erhöhen kann, wenn der Nanodiamantanteil an dem Dentalmaterial kleiner 25 Gew.-%, vorzugsweise 2-25 Gew.-%, weiter vorzugsweise 3-25 Gew.-%, weiter vorzugsweise 5-25 Gew.-%, weiter vorzugsweise 7-23 Gew.-%, weiter vorzugsweise 9-21 Gew.-%, weiter vorzugsweise 10-20 Gew.-%, weiter vorzugsweise 15-20 Gew.-%, weiter vorzugsweise 16-19 Gew. %, weiter vorzugsweise 17-19 Gew.-%, besonders bevorzugt 17,5-18,5 Gew.-% beträgt.

Erfindungsgemäß sind die Nanodiamanten in die organische Harzmatrix eindispergiert. Weitere Füllstoffe und Additive werden anschließend in die so erhaltene Nanodispersion eingerührt.

Der Gesamtgehalt an Nanopartikeln (einschließlich Nanodiamant) im polymerisierbaren Dentalmaterial ist vorzugsweise nicht größer als 25 Gew.-%, vorzugsweise kleiner 23 Gew.-%, weiter vorzugsweise kleiner 21 Gew.-%, weiter vorzugsweise 15-20 Gew.-%., weiter vorzugsweise 16-19 Gew.-%, weiter vorzugsweise 17-19 Gew.-%, besonders bevorzugt 17,5-18,5 Gew.-%.

Erfindungsgemäß bevorzugt besteht die organische Phase aus polymerisierbaren Monomeren, ausgewählt aus der Gruppe bestehend aus Acrylsäureester und Methacrylsäureester. Die Harzmatrix kann aber auch aus Monomeren bestehen, die ringöffnend oder ionisch polymerisieren. Als organische Phase wird erfindungsgemäß eine Verbindung oder eine Mischung mehrerer Verbindungen eingesetzt, die radikalisch und/oder kationisch und/oder anionisch polymerisierbare Gruppen und/oder Gruppen, die eine Aushärtung über eine Kondensations- und/oder Additionsreaktion und/oder über eine Säure-Base-Reaktion erlauben, enthält. Die Verbindungen bestehen aus einem Phospazen-basierten, Silizium-basierten oder organischen (Kohlenstoff-basierten) Grundgerüst und mindestens einer funktionellen Gruppe, die an dieses Grundgerüst gebunden ist und die eine über eine radikalische und/oder kationische und/oder anionische Polymerisationsreaktion und/oder über eine Kondensations- und/oder Additionsreaktion und/oder über eine Säure-Base-Reaktion ablaufende Aushärtung erlaubt. Bei diesen funktionellen Gruppen handelt es sich bevorzugt um Acrylat-, Methacrylat-, Cyanacrylat-, Acrylamid-, Methacrylamid-, Vinyl-, Allyl-, Epoxid-, Oxetan-, Vinylether-, Amino-, Säure-, Säureester-, Säurechlorid-, Phosphat-, Phosphonat-, Phosphit-, Thiol-, Alkohol-und/oder Isocyanatgruppen. Die Säure-, Säureester- und Säurechloridgruppen können z. B. von Carbonsäuren, Phosphorsäuren, Phosphonsäuren oder Sulfonsäuren abgeleitet sein.

Das Grundgerüst kann linear, verzweigt, zyklisch, dendritisch und/oder hyperverzweigt ("hyperbranched") aufgebaut sein. Beim Grundgerüst kann es sich um eine monomere, oligomere oder polymere Struktur handeln. Die chemische Struktur des Grundgerüsts ist aus aliphatischen, zykloaliphatischen, heterozyklischen, aromatischen und/oder heteroaromatischen Segmenten aufgebaut. Innerhalb der aliphatischen, zykloaliphatischen, heterozyklischen, aromatischen und/oder heteroaromatischen Segmente können eine oder mehrere funktionelle Gruppen enthalten sein, z. B.-O-, -S-, -SO-, -SO₂-, -NR¹-, -PR¹-, -P(OR¹)-, -POR¹-, -PO(OR¹)-, -O-PO(OR¹)-O-, - CO-, -CO₂-, O-CO-O-, -COS-, -CS₂-, -C=N-, -N=C=N-, -CO(NR¹)-, O-CO-NR¹-, -NR¹-CO-NR²-, -SiR¹R²- und/oder -SiR¹R²-O-, wobei R¹ = H oder jedbeliebiger organische Rest, bevorzugt ein unsubstituierter oder substituierter Alkyl- bzw. ArylRest R² = H oder jedbeliebiger organischer Rest, bevorzugt ein unsubstituierter oder substituierter Alkyl- bzw. ArylRest; gleich oder verschieden von R¹.

Sind aliphatische Segmente im Grundgerüst enthalten, so sind diese bevorzugt von substituierten (z. B. halogenierten) und unsubstituierten Alkyl- und Alkenyl-Verbindungen, Ethern, Estern, Carbonaten, Urethanen, Polyethern, Polyestern, Polycarbonaten und Polyurethanen abgeleitet.

Sind zykloaliphatische Segmente im Grundgerüst enthalten, so sind diese bevorzugt von substituierten (z. B. halogenierten) und unsubstituierten Cycloalkanen (wie z. B. Cyclohexan und seine Derivate), Spiranen (wie z. B. Spiro[3.3]heptan) und bi- und polyzyklischen Kohlenwasserstoffen (wie z.B. Decalin, Norbornan, Norcaran, Pinan, Adamantan, Twistan und Diamantan) abgeleitet.

Sind heterozyklische Segmente im Grundgerüst enthalten, so sind diese bevorzugt von substituierten (z. B. halogenierten) und unsubstituierten Cyclodextrinen, Morpholinen und Iminooxadiazindionen abgeleitet.

Sind aromatische Segmente im Grundgerüst enthalten, so sind diese bevorzugt von substituierten (z. B. halogenierten) und unsubstituierten Benzenen (wie z.B. Benzol, Toluol, Phenol, Anilin und Biphenyl) und kondensierten aromatischen Ringsystemen (wie z. B. Inden, Fluoren, Naphthalin, Acenaphthen, Anthracen, Phenanthren, Naphthacen, Pyren und Chrysen) abgeleitet.

Sind heteroaromatische Segmente im Grundgerüst enthalten, so sind diese bevorzugt von substituierten (z. B. halogenierten) und unsubstituierten Pyrrolen, Furanen, Thiophenen, Indolen, Benzofuranen, Benzothiophenen, Dibenzofuranen, Dibenzothiophenen, Pyrazolen, Imidazolen, Pyridinen, Pyranen, Thiopyranen und Chinolinen abgeleitet.

Als organische Phase kann auch eine flüssigkristalline Verbindung oder eine Mischung mehrerer Verbindungen, von denen mindestens eine flüssigkristallin ist, eingesetzt werden, die radikalisch und/oder kationisch und/oder anionisch und/oder ringöffnend polymerisierbare Gruppen und/oder Gruppen, die eine Aushärtung über eine Kondensations-und/oder Additionsreaktion und/oder über eine Säure-Base-Reaktion erlauben, enthält.

Eine weitere bevorzugte Option ist die Verwendung einer fluoridionenfreisetzenden Verbindung oder einer Mischung mehrerer Verbindungen, von denen mindestens eine Fluoridionen freisetzen kann, als organisches Bindemittel, das zusätzlich radikalisch und/oder kationisch und/oder anionisch und/oder ringöffnend polymerisierbare Gruppen und/oder Gruppen, die eine Aushärtung über eine Kondensations-und/oder Additionsreaktion und/oder über eine Säure-Base-Reaktion erlauben, enthält.

Als organische Phase werden besonders bevorzugt Acrylate, Methacrylate, Acrylamide, Methacrylamide, Vinylether, Epoxide, Oxetane, Spiroorthocarbonate, Spiroorthoester, bizyklische Orthoester, bizyklische Monolactone, bizyklische Bislactone, zyklische Carbonate, zyklische Acetale, Allylsulfide, Vinylzyklopropane, organische Phosphate, organische Phosponate, organische Phosphite oder eine Kombination aus diesen Verbindungen eingesetzt. Die Allgemeinheit nicht einschränkend sind nachfolgend einige Beispiele genannt: Methyl(meth)acrylat, Ethyl(meth)acrylat, n- oder i-Propyl(meth)acrylat, n-, i- oder tert.-Butyl(meth)acrylat, Hexyl(meth)acrylat, 2-Ethylhexyl(meth)acrylat, Cyclohexyl(meth)acrylat, Isobornyl(meth)acrylat, Hydroxyethyl(meth)acrylat, Hydroxypropyl(meth)acrylat, Phosphorsäureester von Hydroxyethyl(meth)acrylat bzw. Hydroxypropyl(meth)acrylat, (Meth)acrylsäure, Malonsäure-mono-(meth)acrylatester, Bersteinsäure-mono-(meth)acrylatester, Maleinsäure-mono-(meth)acrylatester, Glycerin(meth)acrylat, Glycerin(meth)acrylatester, Glycerindi(meth)acrylat, Glycerindi(meth)acrylatester (wie z. B. Glycerindi(meth)acrylatsuccinat), 4-(Meth)acryloyloxyethyltrimellithsäure, Bis-4,6- bzw. Bis-2,5-(Meth)acryloyloxyethyltrimellithsäure, 2-(((Alkylamino)-carbonyl)oxy)ethyl-(meth)acrylate, Allyl(meth)acrylat, Butandioldi(meth)acrylat, Hexandioldi(meth)acrylat, Decandioldi(meth)acrylat, Dodecandioldi(meth)acrylat, Ethylenglykoldi(meth)acrylat, Diethylenglykoldi(meth)acrylat, Triethylenglykoldi(meth)acrylat, Tetraethylenglykoldi(meth)acrylat, Polyethylenglykoldi(meth)acrylate, Glycerindi(meth)acrylat, Glycerolpropoxytri(meth)acrylat, Trimethylolpropantri(meth)acrylat, ethoxylierte und/oder propoxylierte Trimethylolpropantri(meth)acrylate, Pentaerythrittetra(meth)acrylat, Dipentaerythritolhexa(meth)acrylat, Bisphenol-A-di(meth)acrylat, ethoxylierte und/oder propoxylierte Bisphenol-A-di(meth)acrylate, 2,2-Bis-4-(3-(meth)acryloxy-2-hydroxy-propoxy)-phenylpropan und davon abgeleitete Verbindungen, Chlor- und Bromphosphorsäureester des Bisphenol-A-glycidyl(meth)acrylats, Urethan(meth)acrylate (wie z. B. 7,7,9-Trimethyl-4,13-dioxo-3,14-dioxa-5,12-diazahexadecan-1,16-dioxy-dimethacrylat), Polyesterurethan(meth)acrylate, Polyester(meth)acrylate, Polycarbonat(meth)acrylate, Polyamid(meth)acrylate, Polyimid(meth)acrylate, Phosphazen(meth)acrylate und Siloxan(meth)acrylate; Ethylvinylether, n- oder i-Propylvinylether, n-, i- oder tert.-Butylvinylether, Hexylvinylether, Octylvinylether, Cyclohexylvinylether, Cyclohexyl-3,4-epoxy-1-methylvinylether, Dimethanol-cyclohexyl-monovinylether, 1,4-Dimethanolcyclohexyl-divinylether, Propandioldivinylether, Butandioldivinylether, Hexandioldivinylether, Octandioldivinylether, Decandiolvinylether, Ethylenglykoldivinylether, Diethylenglykoldivinylether, Triethylenglykoldivinylether, Triethylenglykol-monovinylether-mono(meth)acrylsäureester, Polyethylenglykoldivinylether, Tripropylenglykoldivinylether, Glycerintrivinylether, Pentaerythrittetravinylether, 7,7,9-Trimethyl-4,13-dioxo-3,14-dioxa-5,12-diazahexadecan-1,16-dioxydivinylether, Bisphenol-A-divinylether, ethoxylierte und/oder propoxylierte Bisphenol-A-divinylether, Polyestervinylether, Polycarbonatvinylether, Polyacrylatvinylether, Polyamidvinylether, Polyimidvinylether, Polyurethanvinylether, Phosphazenvinylether und Siloxanvinylether; Alkylglycidylether, Glycidol, Glycidyl(meth)acrylat, Dipentendioxid, 1,2-Epoxyhexadecan, Bis-(3,4-epoxycyclohexyl)-adipat, Vinylcyclohexenoxide, Vinylcyclohexendioxide, Epoxycyclöhexancarboxylate (wie z. B. 3,4-Epoxycyclohexylmethyl-3,4-epoxycyclohexen-carboxylat), Butandioldiglycidylether, Hexandioldiglycidylether, Dodecandioldiglycidylether, Diglycidylether der Polyethylenglykole und der Polypropylenglykole, Diglycidylether von substituierten (z. B. halogenierten) und unsubstituierten Bisphenolen (wie z. B. Bisphenol-A, Bisphenol-C und Bisphenol-F), Resorcindiglycidylether, Trimetylolethantriglycidylether, Trimetylolpropantriglycidylether, Polybutadienpolyepoxide, Polyesterepoxide, Polycarbonatepoxide, Polyacrylatepoxide, Polyamidepoxide, Polyimidepoxide, Polyurethanepoxide, Phosphazenepoxide und Siloxanepoxide; 3,3-disubstituierte Oxetane und Dioxetane (wie z.B. 3-Ethyl-3-(2-hydroxyethyl)-oxetan); (trans/trans)-2,3,8,9-Di(tetramethylen)-1,5,7,11-tetraoxaspira-[5.5]-undecan; substituierte 1,3-Dioxolane (wie z. B. 2-Phenyl-4-methylen-1,3-dioxolan); difunktionelle 6-Methylen-1,4-dithiepane sowie die Umsetzungsprodukte von nukleophilen (Meth)acrylaten wie z. B. 2-Hydroxyethyl(meth)acrylat oder Glycerin(meth)acrylatestern mit reaktiven Phosphorsäure-, Phosphonsäure- oder Phosphinsäurederivaten wie z. B. P₂O₅ POCl₃ oder PCl₃. Zur Aushärtung des erfindungsgemäßen Dentalmaterials wird bevorzugt ein Initiator bzw. mehrere Initiatoren und optional ein Coinitiator bzw. mehrere Coinitiatoren in das erfindungsgemäße Dentalmaterial eingearbeitet. Dabei können Initiator bzw. Initiatoren und Coinitiator bzw. Coinitiatoren zusammen in einer Komponente und/oder getrennt in zwei oder mehreren Komponenten enthalten sein. Das erfindungsgemäße Dentalmaterial kann somit thermisch, chemisch, photochemisch, d. h. durch Bestrahlung mit UV- und/oder sichtbarem Licht, und/oder durch Reaktion mit der Mund- und/oder Luftfeuchte ausgehärtet werden.

Die hier verwendbaren Initiatoren können z. B. Photoinitiatoren sein. Diese sind dadurch charakterisiert, dass sie durch Absorption von Licht im ultravioletten oder sichtbaren Bereich (Wellenlängenbereich von 300 nm bis 700 nm, bevorzugt von 350 nm bis 600 nm und besonders bevorzugt von 380 nm bis 500 nm) und optional durch die zusätzliche Reaktion mit einem oder mehreren Coinitiatoren die Aushärtung des Materials bewirken können. Bevorzugt werden hier Phosphinoxide, Bezoinether, Benzilketale, Acetophenone, Benzophenone, Thioxanthone, Bisimidazole, Metallocene, Fluorone, α- Dicarbonylverbindungen, Aryldiazoniumsalze, Arylsulfoniumsalze, Aryliodoniumsalze, Ferroceniumsalze, Phenylphosphoniumsalze oder eine Mischung aus diesen Verbindungen eingesetzt.

Besonders bevorzugt werden Diphenyl-2,4,6-trimethylbenzoyl-phosphinoxid, Benzoin, Benzoinalkylether, Benzildialkylketale, α-Hydroxy-acetophenon, Dialkoxyacetophenone, α-Aminoacetophenone, i-Propylthioxanthon, Campherchinon, Phenylpropandion, 5,7-Diiodo-3-butoxy-6-fluoron, (eta-6-Cumol)(eta-5-cyclopentadienyl)eisen-hexafluorophosphat, (eta-6-Cumol)(eta-5-cyclopentadienyl)eisentetrafluoroborat, (eta-6-Cumol)(eta-5-cyclopentadienyl)eisen-hexafluoroantimonat, substituierte Diaryliodoniumsalze, Triarylsulfoniumsalze oder eine Mischung aus diesen Verbindungen eingesetzt.

Als Coinitiatoren für eine photochemische Aushärtung werden bevorzugt tertiäre Amine, Borate, organische Phosphite, Diaryliodoniumverbindungen, Thioxanthone, Xanthene, Fluorene, Fluorone, α-Dicarbonylverbindungen, kondensierte Polyaromaten oder eine Mischung aus diesen Verbindungen eingesetzt. Besonders bevorzugt werden N,N-Dimethyl-p-toluolidin, N,N-Dialkyl-alkyl-aniline, N,N-Dihydroxyethyl-p-toluidin, 2-Ethylhexyl-p-(dimethyl-amino)-benzoat, Butyrylcholin-triphenylbutyl-borat oder eine Mischung aus diesen Verbindungen eingesetzt.

Als Initiatoren können auch sogenannte thermische Initiatoren eingesetzt werden, die durch die Aufnahme von thermischer Energie bei erhöhter Temperatur die Aushärtung des Materials bewirken können. Hierbei werden bevorzugt anorganische und/oder organische Peroxide, anorganische und/oder organische Hydroperoxide, α,α'-Azo-bis(isobutyroethylester), α,α'-Azo-bis(isobutyronitril), Benzpinakole oder eine Mischung aus diesen Verbindungen eingesetzt. Besonders bevorzugt werden Diacylperoxide wie z. B. Benzoylperoxid oder Lauroylperoxid, Cumolhydroperoxid, Benzpinakol, 2,2'-Dimethylbenzpinakol oder eine Mischung aus diesen Verbindungen eingesetzt.

Für eine chemische Aushärtung bei Raumtemperatur wird i. a. ein Redoxinitiatorsystem verwendet, das aus einem bzw. mehreren Initiatoren und einem als Aktivator dienenden Coinitiator bzw. Coinitiatoren besteht. Aus Gründen der Lagerstabilität werden Initiator bzw. Initiatoren und Coinitiator bzw. Coinitiatoren in räumlich voneinander getrennten Teilen des erfindungsgemäßen Dentalmaterials eingearbeitet, d. h. es liegt ein mehrkomponentiges, bevorzugt ein zweikomponentiges Material vor. Als Initiator bzw. Initiatoren werden bevorzugt anorganische und/oder organische Peroxide, anorganische und/oder organische Hydroperoxide, CH-acide Verbindungen wie beispielsweise Barbitursäurederivate und deren Salze, Malonylsulfamide, Protonensäuren, Lewis- oder Broensted-Säuren bzw. Verbindungen, die solche Säuren freisetzen, Carbeniumionen-Donatoren wie z. B. Methyltriflat oder Triethylperchlorat oder eine Mischung aus diesen Verbindungen und als Coinitiator bzw. als Coinitiatoren bevorzugt tertiäre Amine, Schwermetallverbindungen, insbesondere Verbindungen der 8. und der 9. Gruppe des Periodensystems ("Eisen- und Kupfergruppe"), Verbindungen mit ionogen gebundenen Halogenen oder Pseudohalogenen wie z. B. quarternäre Ammoniumhalogenide, schwache Broenstedt-Säuren wie z. B. Alkohole und Wasser oder eine Mischung aus diesen Verbindungen eingesetzt.

In dem erfindungsgemäßen Dentalmaterial kann auch jede denkbare Kombination der oben beschriebenen Initiatoren und Coinitiatoren enthalten sein. Ein Beispiel hierfür sind sogenannte dualhärtende Dentalmaterialien, die sowohl Photoinitiatoren und optional die entsprechenden Coinitiatoren für eine photochemische Aushärtung als auch Initiatoren und entsprechende Coinitiatoren für eine chemische Aushärtung bei Raumtemperatur enthalten.

Gemäß einer bevorzugten Ausführungsform der Erfindung weisen die Nanodiamanten einen mittleren Primärteilchen-Durchmesser von 0,5-100 nm, vorzugsweise 1-60 nm, weiter vorzugsweise 1-40 nm, weiter vorzugsweise 1-20 nm, weiter vorzugsweise 2-8 nm, weiter vorzugsweise 3-6 nm, weiter vorzugsweise 3,5-5,5 nm auf. Derartige Nanodiamanten lassen sich durch verschiedenste aus dem Stand der Technik bekannte Verfahren gewinnen und sind beispielsweise ausführlich beschrieben in O.A. Shenderova; V.V. Zhirnov; D.W. Brenner: "Carbon Nanostructures", Critical Reviews in Solid State and Materials Sciences 27 (3/4) [2002] S. 227-356, insbesondere Seiten 266 ff. oder der eingangs erwähnten WO-A-2005/097045. Beispielhaft verwiesen sei auf die Herstellung von Nanodiamanten auf folgende Arten:
- Herstellung von 0-dimensionalen Nanodiamanten durch Reaktion in der Gasphase (Niederdrucksynthese im Microwellen Plasma Reaktor durch Umsetzung von CH₂Cl₂/O₂-Gemischen: M.Frenklach et al., J. Appl. Phys. 66 [1989] S. 395 - 399; ggf unter Zusatz von B₂H₂: M. Frenklach et al., Appl. Phys.Lett. 59 [1991] S. 546 - 548);
- Herstellung von Detonationsnanodiamanten (UDD = ultradispersed diamond entsteht dadurch, dass Sprengstoffe unter Luftabschluß zur Detonation gebracht werden und aus deren Bestandteilen unter hohem Druck Nanodiamant entsteht);
- Herstellung von Nanodiamanten durch Schockwellensynthese (es wird eine Schockwelle erzeugt, die dann auf Kohlenstofftargets unter Bildung von Nanodiamant einwirkt: Y.Q.Zhul et al., Chem. Phys. Lett. 287 [1998] S. 689 - 693);
- Herstellung von Nanodiamanten durch Einwirkung von Strahlung auf Kohlenstoffspezies (Einwirkung von CVD Plasma von CH₄/H₂: Y. Lifshitz et al.: Science 297 [2002] S. 1531 - 1533 oder von Ionenstrahlung unter Verwendung von Kohlenwasserstoffen, Wasserstoff oder Argon: H. Yusa, Diam. Relat. Mat. 11 [2002] S. 87 - 91 auf amorphe Kohlenstoffmatrixes an Siliziumoberflächen);
- Herstellung von Nanodiamanten durch Ionenbombardment von SiC (Siliziumcarbid) mit C (60 keV, 900°C: F. Li et al., Appl. Phys. Lett. 77 [200] S. 3161 - 3163) oder von fein verteilten polykristallinen Graphit mit Krypton-Ionen (20°C, 350 MeV: T.L.Daulton et al., Nicl. Inst. Meth. Phys. Res. B175 [2001] S. 12 - 20);
- Herstellung von Nanodiamanten durch Umwandlung von Kohlenstoffspezies im Elektronenstrahl (1,2 MeV, 900 K: P. Banhart et al., Nature 382 [1996] S. 433);
- Herstellung von Nanodiamanten durch aus synthetisch hergestellten oder natürlich vorkommenden groberen Diamantpulvern gegebenenfalls nach Mahlung ausgesiebt, durch Klassierung oder andere geeignete Trennverfahren abgetrennt; und
- Diamant-nanorods (durch selektives Ätzen von CVD Diamantfilmen: E.-S. Baik et al. J. Mat. Res. 15 [200] S. 923 - 926) und Nanofasern (durch Laserabtragung von UDD-Pellets: A. Koscheev, Ximia zizn 1 [1999] S. 14 - 16).

Wie bereits erwähnt finden sich nähere Informationen zu den genannten Verfahren und zu solchen, die zu polykristallinen Naondiamantpulvern führen, beispielsweise in in O. A. Shenderova et al., "Carbon Nanostructures", Critical Reviews in Solid State and Materials Sciences 27 (3/4) [2002] S. 249ff.

Die Herstellung transparenter bzw. transluzenter Nanodiamantpulver kann von Vorteil sein, wenn die daraus hergestellten Dentalmaterialien transluzent sein sollen. So kann es beispielsweise erwünscht sein, dass Nanodiamanten enthaltende Fissurenversiegeler transluzent sind, um als solche nicht auf der Zahnoberfläche visuell wahrgenommen zu werden. Außerdem sind transluzente Füllstoffe erforderlich, um Materialien hoher Transparenz beispielsweise für den Schmelzersatz oder Materialien in sehr helle Farben herstellen zu können.

Das erfindungsgemäße Dentalmaterial kann in der anorganischen Phase dental übliche Füllstoffe enthalten. Bei den erfindungsgemäß eingesetzten Füllstoffen handelt es sich bevorzugt um nano- und/oder mikroskalige Füllstoffe, vorzugsweise um Glaspulver, Glaskeramikpulver, Metall-, Halbmetall- oder Mischmetalloxide, Silikat-, Nitrid-, Sulfat-, Titanat-, Zirkonat-, Stannat-, Woframat-, Siliciumdioxid-Verbindungen oder eine Mischung aus diesen Verbindungen oder sphärische Füllstoffe, Quarzpulver oder eine Mischung aus diesen Pulvern oder gefüllte oder ungefüllten Splitterpolymerisate und/oder Perlpolymerisate.

Bei den erfindungsgemäß eingesetzten nano- oder mikroskaligen Füllstoffen handelt es sich besonders bevorzugt um Dentalgläser, Siliziumdioxid, Aluminiumoxid, Zirkondioxid, Titandioxid, Zinkoxid, Zinndioxid, Ceroxid, Aluminium-Silizium-Oxide, Silizium-Zink-Oxide, Silizium-Zirkon-Oxide, Eisenoxide und deren Mischungen mit Siliziumdioxid, Indiumoxide und deren Mischungen mit Siliziumdioxid und/oder Zinndioxid, Bornitrid, Strontiumsulfat, Bariumsulfat, Strontiumtitanat, Bariumtitanat, Natriumzirkonat, Kaliumzirkonat, Magnesiumzirkonat, Calciumzirkonat, Strontiumzirkonat, Bariumzirkonat, Natriumwolframat, Kaliumwolframat, Magnesiumwolframat, Calciumwolframat, Strontiumwolframat und/oder Bariumwolframat.

Der Füllstoff kann gemäß einer bevorzugten Ausführungsform der Erfindung ein oberflächenmodifizierter Füllstoff, vorzugsweise ein organisch oberflächenmodifizierter Füllstoff sein. Der Füllstoff kann nach seiner Oberflächenmodifikation, beispielsweise einer Silanisierung, auf seiner Oberfläche funktionelle Gruppen, beispielsweise reaktive Methacrylatgruppen besitzen, die mit den Monomeren chemisch, vorzugsweise radikalisch, reagieren können oder eine hohe Affinität zu der aus den Monomeren gebildeten Polymermatrix aufweisen.

Das erfindungsgemäße Dentalmaterial kann zur Einstellung bestimmter Eigenschaften zusätzlich so genannte Additive bzw. Modifikatoren enthalten. Die Allgemeinheit nicht einschränkend sind nachfolgend einige Beispiele genannt: anorganische und/oder organische Farbpigmente bzw. Farbstoffe, Stabilisatoren (wie z.B. substituierte und unsubstituierte Hydroxyaromaten, Tinuvine, Terpinene, Phenothiazin, sogenannte HALS - Hindered Amine Light Stabilizers - und/oder Schwermetallfänger wie EDTA), Weichmacher (wie z. B. Polyethylenglykole, Polypropylenglykole, ungesättigte Polyester, Phthalate, Adipate, Sebacate, Phosphorsäureester, Phosphonsäureester und/oder Zitronensäureester), ionenabgebende Substanzen, insbesondere solche die Fluoridionen freisetzen, wie z. B. ionenfreisetzende Dentalgläser (Reaktivgläser), Natriumfluorid, Kaliumfluorid, Yttriumfluorid, Ytterbiumfluorid und/oder quartäre Ammoniumfluoride), bakterizide oder antibiotisch wirksame Substanzen (wie z. B. Chlorhexidin, Pyridiniumsalze, Penicilline, Tetracycline, Chloramphenicol, antibakterielle Makrolide und/oder Polypeptid-Antibiotika) und/oder Lösungsmittel (wie z. B. Wasser, Aceton, Ethanol, i-Propanol, Butanon und/oder Essigsäureethylester).

Das erfindungsgemäße Dentalmaterial kann für die prothetische, konservierende und präventive Zahnheilkunde sowie im zahntechnischen Labor verwendet wenden. Ohne Anspruch auf Vollständigkeit seien stellvertretend einige Anwendungsbeispiele genannt: Füllungsmaterial, Unterfüllungsmaterial, Stumpfaufbaumaterial, Befestigungsmaterial, provisorisches und permanentes Kronen- und Brückenmaterial, Bonding-Materialien, zahntechnischer Werkstoff zur Herstellung von Inlays, Onlays, Verblendschalen, künstlichen Zähnen, Modellmaterialien und Fissuren- und Wurzelkanal-Versiegelungsmaterial.

Die Erfindung wird nachfolgend ohne Einschränkung der Allgemeinheit anhand von Ausführungsbeispielen erläutert.

### Beispiel 1: Charakterisierung des in Beispiel 3 verwendeten Nanodiamants

Für die Herstellung der Komposite gemäß Beispiel 3 wurde Nanodiamantpulver der Fa. New Metals And Chemicals Corporation, Ltd. (Kyobashi TD, Kyobashi, Chuo-Ku, Tokyo) eingesetzt.

Das Diamantpulver wurde nach dem Detonationsverfahren aus 2,4,6-Trinitrotoluen (TNT) und 1,3,5-Trinitro-1,3,5-triazinan (Hexogen) hergestellt und besitzt eine sphärische bis ellipsoide Teilchenform mit einer Primärteilchengröße zwischen 3nm und 10 nm, die mittels Röntgendiffraktometrie (X-Ray-Diffraction oder XRD), dynamischer Lichtstreuung und hochauflösender Transmissionselektronenmikroskopie(High Resolution Transmission Electron Microscopy oder HRTEM) ermittelt wurde. Die spezifische Oberfläche liegt zwischen 278 und 335 m²/g. Die mittels Elementaranalyse bestimmte chemische Zusammensetzung beträgt durchschnittlich 91% Kohlenstoff, 2% Wasserstoff, 5% Sauerstoff, und 2% Stickstoff. Elektronenspinresonanz (Electron Paramagnetic Resonance oder EPR)-Spektroskopieuntersuchungen zeigen, dass der größte Teil des Stickstoffs in das Diamantgitter eingebaut ist und sich der Sauerstoff und Wasserstoff zusammen mit den Anteilen des Stickstoffs als Sauerstoff-, Stickstoff- und Wasserstoff-haltige funktionelle Gruppen auf der Oberfläche der Diamantkristallite (z.B.: -OH, -C=O, -COOH, -C-O-C, -CN gemäß den Angaben des Herstellers) befinden. Die Dichte des Nanodiamantpulvers liegt zwischen 3,05 und 3,12 g/ml.

### Beispiel 2: Charakterisierung des in Beispiel 3 in den Komposits verwendeten Grundharzes

Als Grundharz für die Herstellung der Komposits gemäß Beispiel 3 wurde eine Harzmischung mit folgenden Komponenten verwendet:
31,73 Gew.-% Bis GMA,
31,73 Gew.-% ethoxyliertes Bis-Phenol-A-DMA,
18,23 Gew.-% TEDMA,
18,23 Gew.-% Trimethylolpropan-trimethacrylat,
0,08 Gew.-% 2,6-Di-tert. Butyl-4-methyl-phenol.

### Beispiel 3 : Herstellung und Untersuchung der mechanischen Eigenschaften von Kompositmaterialien ohne und mit Nanodiamant

Es wurden die fünf nachfolgenden unterschiedlichen Kompositmaterialien
- Komposit 1:: Komposit ohne Nanodiamant und mit 1,92 Gew.-% Aerosil als Nanopartikel;
- Komposit 2:: Komposit ohne Nanodiamantund mit 18,48 Gew.-% Aerosil als Nanopartikel;
- Komposit 3:: Komposit mit 1,21 Gew.-% Nanodiamant und 1,92 Gew.-% Aerosil als Nanopartikel;
- Komposit 4:: Komposit mit 15,73 Gew.-% Nanodiamantund 4,61 Gew.-% Aerosil als Nanopartikel;
- Komposit 5:: Komposit mit 16,56 Gew.-% Nanodiamant und 1,92 Gew.-% Aerosil als Nanopartikel;
in folgender Art und Weise hergestellt:

Zunächst wurden das Nanodiamantpulver in das Grundharz der Basispasten ("B" in den nachfolgenden Tabellen) und Initatorpasten ("I" in den nachfolgenden Tabellen) gemäß Beispiel 2 mit den weiteren Komponenten, wie in den nachfolgenden Tabellen im Einzelnen aufgeführt, eindispergiert. Dazu wurde ein Dispergierrad mit einem Durchmesser von 40 mm verwendet. Das flüssige Medium wurde in einem mit Wasser gekühlten (doppelwandigen) Edelstahlgefäß (T < 40°C, ∅ 79 mm) vorgelegt und das Nanodiamantpulver gemäß Beispiel 1 portionsweise zugegeben. Die Gesamtdispergierzeit betrug 4 Stunden. Dabei wurde zunächst bei Drehzahlen von 5000-6000 1/min (10,5 m/s - 12,6 m/s) das Nanodiamantpulver eingearbeitet (15-30 min). Anschließend wurde die Drehzahl auf 10000 1/min (21 m/s) gesteigert. Mit Zunahme der Viskosität der Nanodispersion wurde die Drehzahl so angepasst, dass ein Freilauf der Dispergierscheibe vermieden wurde.

Anschließend wurde die Nanodispersion in den Behälter einer Knetmaschine überführt und zuerst die Komponenten eines Startersystems eingearbeitet. Danach wurde eine geringe Menge Aerosil, das der Einstellung bestimmter handlingEigenschaften diente eingerührt. Schließlich wurden das Dentalglas und gegebenenfalls weitere Additive eingearbeitet. Dabei wurde der Füllstoffgehalt so bemessen, dass fließfähige (flowable) Komposite resultierten und die Druckfestigkeit der aus Basis- und Initiatorpasten im Verhältnis 1 : 1 angemischten Komposite (B+I) erfolgte nach einem aus der DIN 13902 vom Dezember 1972 für Silikatzemente abgeleiteten Prüfverfahren. Dafür wurden die Abmessungen der Prüfkörper an die höheren Druckfestigkeiten harzgebundener Komposite angepaßt. Zur Herstellung der Probekörper wurde eine teilbare aus nicht rostendem Stahl gefertigte Form mit 6 polierten zylindrischen Bohrungen mit einer Höhe von h = 4,00±0,02mm und einem Durchmesser von d = 2,00±0,01mm verwendet. Die Form wurde auf einen Objektträger, der mit Hostafan-Folie bedeckt war, gelegt. Zuvor wurde die Form mit einem Trennmittel (Silikonspray) eingesprüht. Das Material wurde von Hand im Verhältnis 1 : 1 angemischt und blasenfrei in die Bohrungen der Form eingebracht. Die Form wird erneut mit einem Stück Folie und einem Objektträger bedeckt. Das überschüssige Material wurde herausgepreßt und die Objektträger mit einer Klemme fixiert. Dann wurde die gefüllte Form in einen Drucktopf (Polymax-5 der Fa. Dreve) gegeben und das Material 10 min. bei 40°C und 6 bar ausgehärtet. Anschließend wird die Form entnommen, Objektträger und Folie entfernt und mit Schleifpapier P600 nass von überstehendem Material befreit. Danach werden die Prüfkörper entformt und in ein Gläschen mit destilliertem Wasser 23 Stunden bei 37°C gelagert. Anschließend werden die Prüfkörper auf 23°C abgekühlt und visuell auf Beschädigungen untersucht. Schadhafte Prüfkörper werden ausgesondert. Die Messung der Druckfestigkeit erfolgte mit einer Mechanikprüfmaschine Z 010 der Fa. Zwick mit einer 10 kN Meßzelle.

Die folgenden Tabellen zeigen die Rezepturen der Komposite und die gemessenen Druckfestigkeiten.

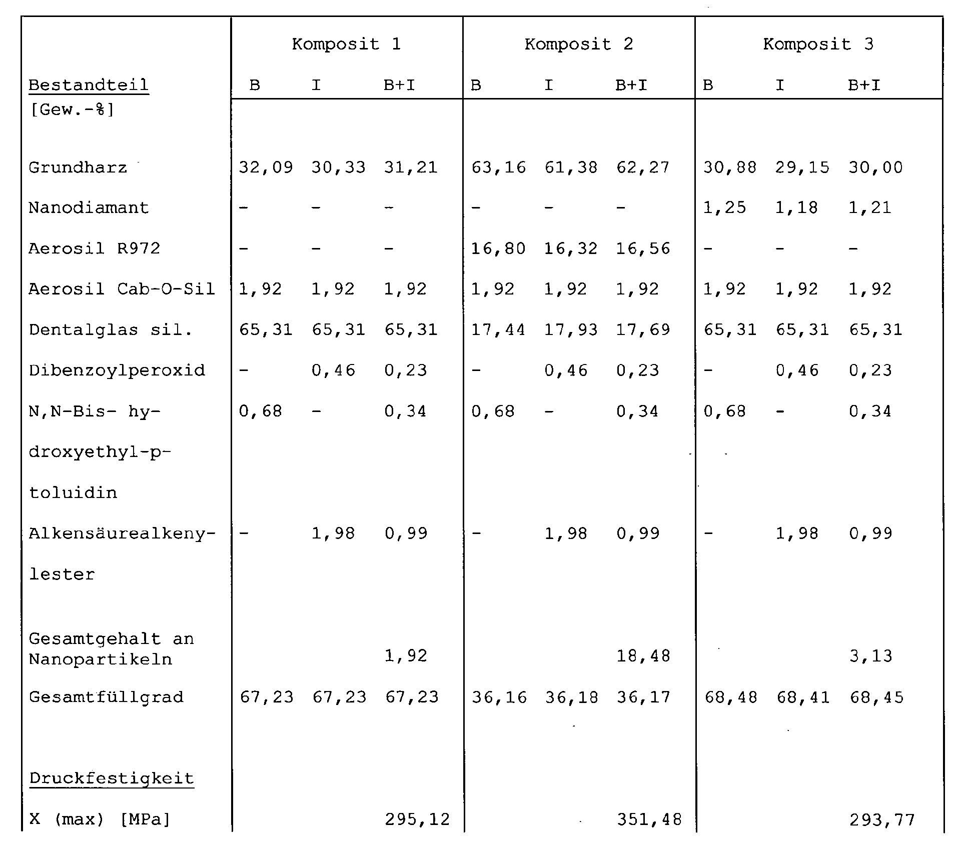

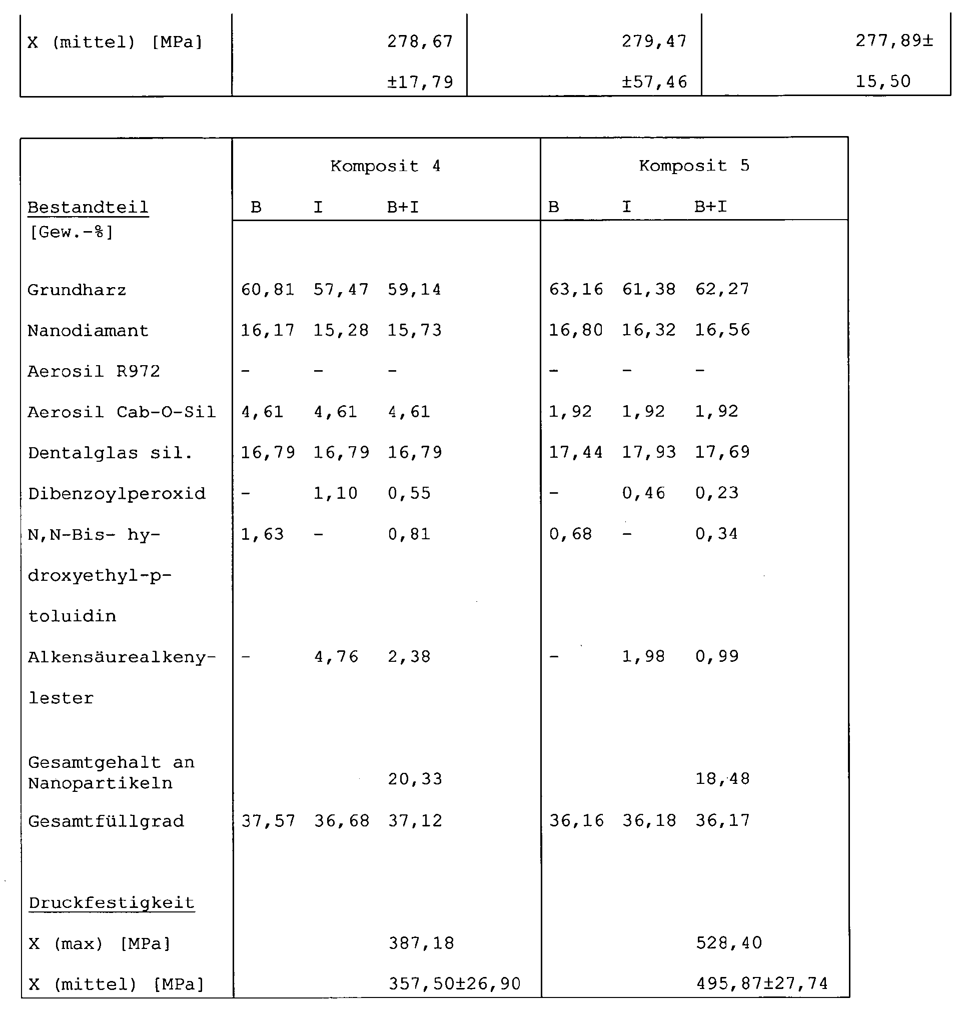

Die Komposite 1 und 2 weisen keine Nanodiamant, Komposit 3 nur einen geringen Anteil von 1,21 Gew.-% Nanodiamant auf. Die gemessenen mittleren Druckfestigkeiten der Komposite 1-3 sind annährend gleich (278,67±17,79 MPa; 279,47±57,46 MPa; 277,09 ± 15,50 MPa).

Im Unterschied dazu weisen die Komposite 4 und 5 mit einem Nanodiamantgehalt von 15,73 Gew.-% und 16,56 Gew.-% eine Erhöhung der mittleren Druckfestigkeit um mehr als 30% (Komposit 4: 357,50±26,90 MPa) bzw. 80% (Komposit 5: 495,87±27,74 MPa) auf.

Das bedeutet, dass mit zunehmenden Nanodiamantgehalt die Druckfestigkeit zunimmt. Andererseits zeigen die Druckfestigkeiten für das Komposit 4 mit 357,50 MPa (Gesamtnanopartikelgehalt von 20,33 Gew.-%) und für das Komposit 5 mit 495,87 MPa (Gesamtnanopartikelgehalt von 18,48 Gew.-%), eine gegenläufige Tendenz, d.h. mit zunehmenden Gesamtnanopartikelgehalt nimmt die Druckfestigkeit ab.

Dass der erfindungsgemäß technische Effekt der Erhöhung der Druckfestigkeit auf den Nanodiamantanteil, und beispielsweise nicht auf den Nanopartikeln des Aerosilanteils beruhen, ergibt sich aus der Gegenüberstellung der Komposite 2 und 5.

Komposit 2 unterscheidet sich von Komposit 5 darin, dass der Nanodiamantanteil durch das Aerosil R972 ersetzt wurde. Das Aerosil R972 der Fa. Degussa besitzt eine spez. Oberfläche von 110 m²/g und eine Primärteilchengröße von 16 nm, deren Oberfläche mit 10% SiCl₂(CH₃)₂ (Dichlordimethylsilan) hydrophobiert ist. Die Druckfestigkeitsmessungen belegen, dass der technische Effekt der Erhöhung der Druckfestigkeit bei dem die Nanodiamanten enthaltenden Komposit 5 vorliegt (495,87 MPa), nicht hingegen bei dem das Aerosil R972 enthaltenden Komposit 2 (279,47 MPa).

## Patentansprüche

1. Polymerisierbares Dentalmaterial mit einer
a) organischen Phase und einer
b) anorganischen Phase,
**dadurch gekennzeichnet, dass** die anorganische Phase Nanodiamant enthält, wobei der Nanodiamantanteil an dem Dentalmaterial kleiner 25 Gew.-% beträgt.

2. Polymerisierbares Dentalmaterial nach Anspruch 1, **dadurch gekennzeichnet, dass** der Nanodiamantanteil an dem Dentalmaterial 2-25 Gew.-%, vorzugsweise 3-25 Gew.-%, weiter vorzugsweise 5-25 Gew.-%, weiter vorzugsweise 7-23 Gew.-%, weiter vorzugsweise 9-21 Gew.-%, weiter vorzugsweise 10-20 Gew.-%, weiter vorzugsweise 15-20 Gew.-%, weiter vorzugsweise 16-19 Gew.-%, weiter vorzugsweise 17-19 Gew.-%, besonders bevorzugt 17,5-18,5 Gew.-% beträgt.

3. Polymerisierbares Dentalmaterial nach einem der Ansprüche 1 oder 2, **dadurch gekennzeichnet, dass** der Gesamtgehalt an Nanopartikeln im polymerisierbaren Dentalmaterial nicht größer als 25 Gew.-%, vorzugsweise kleiner 23 Gew.-%, weiter vorzugsweise kleiner 21 Gew.-%, weiter vorzugsweise 15-20 Gew.-%, weiter vorzugsweise 16-19 Gew.-%, weiter vorzugsweise 17-19 Gew.-%, besonders bevorzugt 17,5-18,5 Gew.-% beträgt.

4. Polymerisierbares Dentalmaterial nach einem der vorangehenden Ansprüche, **dadurch gekennzeichnet, dass** die organische Phase a) eine Verbindung oder eine Mischung mehrerer Verbindungen ist, die radikalisch und/oder kationisch und/oder anionisch und/oder ringöffnend polymerisierbare Gruppen und/oder Gruppen, die eine Aushärtung über eine Kondensations-, Additions- und/oder Säure-Base-Reaktion erlauben, enthält.

5. Polymerisierbares Dentalmaterial nach einem der vorangehenden Ansprüche, **dadurch gekennzeichnet, dass** es zusätzlich einen Initiator bzw. mehrere Initiatoren und optional einen Coinitiator bzw. mehrere Coinitiatoren enthält.

6. Polymerisierbares Dentalmaterial nach einem der vorangehenden Ansprüche, **dadurch gekennzeichnet, dass** die Nanodiamanten einen mittleren Primärteilchen-Durchmesser von 0,5-100 nm, vorzugsweise 1-60 nm, weiter vorzugsweise 1-40 nm, weiter vorzugsweise 1-20 nm, weiter vorzugsweise 2-8 nm, weiter vorzugsweise 3-6 nm, weiter vorzugsweise 3,5-5,5 nm aufweisen.

7. Polymerisierbares Dentalmaterial nach einem der vorangehenden Ansprüche, **dadurch gekennzeichnet, dass** das Nanodiamantpulver auf synthetischem Wege erhalten wurde oder aus natürlichen Quellen stammt.

8. Polymerisierbares Dentalmaterial nach einem der vorangehenden Ansprüche, **dadurch gekennzeichnet, dass** die anorganische Phase des weiteren mindestens einen Füllstoff aufweist, ausgewählt aus der Gruppe bestehend aus: mikro- und/oder nanoskaligen Füllstoffen, vorzugsweise Metall-, Halbmetall- oder Mischmetalloxide, Silikaten, Nitriden, Sulfaten, Titanaten, Zirkonaten, Stannaten, Wolframaten, Siliciumdioxiden oder einer Mischung aus diesen Verbindungen oder sphärischen Füllstoffen, Quarzpulvern, Glaspulvern, Glaskeramikpulvern oder eine Mischung aus diesen Pulvern oder gefüllten oder ungefüllten Splitterpolymerisaten und/oder Perlpolymerisaten.

9. Polymerisierbares Dentalmaterial nach Anspruch 8, **dadurch gekennzeichnet, dass** der Füllstoff ein oberflächenmodifizierter Füllstoff, vorzugsweise ein organisch oberflächenmodifizierter Füllstoff ist.

10. Polymerisierbares Dentalmaterial nach Anspruch 9, **dadurch gekennzeichnet, dass** der oberflächenmodifizierte Füllstoff auf seiner Oberfläche funktionelle Gruppen besitzt, die mit der organischen Phase chemisch, vorzugsweise radikalisch, reagieren können oder eine hohe Affinität zu der aus der organischen Phase gebildeten Polymermatrix haben.

11. Polymerisierbares Dentalmaterial nach Anspruch 8, **dadurch gekennzeichnet, dass** der Füllstoff mit reaktiven Acrylat- und/oder oder Methacrylatgruppen tragendem Silan oberflächenmodifiziert ist.

12. Verwendung des polymerisierbaren Dentalmaterials nach einem der vorangehenden Ansprüche als Füllungsmaterial, Unterfüllungsmaterial, Stumpfaufbaumaterial, Befestigungsmaterial, Bondingmaterial, provisorisches und permanentes Kronen- und Brückenmaterial, zahntechnischen Werkstoff zur Herstellung von Inlays, Onlays, Verblendschalen, künstlichen Zähnen, Modellmaterialien und Fissuren- bzw. Wurzelkanal-Versiegelungsmaterial.

13. Gehärtetes Dentalmaterial, erhältlich aus einem polymerisierbaren Dentalmaterial nach einem der Ansprüche 1 bis 11.
